# EUROPEAN PATENT APPLICATION

(11) **EP 3 564 254 A1**
(43) Date of publication of application: **06.11.2019**
(21) Application number: 17889372.3
(22) Date of filing: 25.12.2017
(51) Int. Cl.: C07K 1/14, C08J 11/08, C12N 15/09

(54) **METHOD FOR RECOVERING PROTEIN**

(30) Priority: 27.12.2016 JP 2016254005
(71) Applicant: Spiber Inc., Yamagata 997-0052 (JP)
(72) Inventor: SEKIYAMA Kazuhide, Tsuruoka-shi Yamagata 997-0052 (JP); OZEKI Akihiko, Tsuruoka-shi Yamagata 997-0052 (JP); OKADA Ryoji, Tsuruoka-shi Yamagata 997-0052 (JP); KOTAKA Koichi, Tsuruoka-shi Yamagata 997-0052 (JP)
(74) Representative: Handley, Matthew Edward
(86) International application number: PCT/JP2017/046394
(87) International publication number: WO 2018/123953

(57) **Abstract**

The present invention relates to a method for recovering protein, which recovers a target protein from a mixture containing the target protein and a material different from the target protein, the method including a dissolution step of dissolving either the target protein or the material by applying pressure while heating to a solution for dissolution containing the mixture and a polar solvent, and a separation step of separating an obtained solution.

SEQUENCE LISTING

<110>  Spiber Inc.

<120>  A method of retrieving a protein

<130>  FP17-0834-00

<160>  11

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
        35                  40                  45

Leu Ala
    50

<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210>  3
<211>  21
<212>  PRT
<213>  Araneus diadematus

<400>  3

Ser Gly Cys Asp Val Leu Val Gln Ala Leu Leu Glu Val Val Ser Ala
1               5                   10                  15

Leu Val Ser Ile Leu
            20

<210>  4
<211>  24
<212>  PRT
<213>  Artificial Sequence

<220>
<223>  His tag and start codon

<400>  4

Met His His His His His His His His His His Ser Ser Gly Ser Ser
1               5                   10                  15

Leu Glu Val Leu Phe Gln Gly Pro
            20

<210>  5
<211>  542
<212>  PRT
<213>  Artificial Sequence

<220>
<223>  PRT204

<400>  5

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln

130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala
    530                 535                 540

<210>  6
<211>  434
<212>  PRT
<213>  Artificial

<220>
<223>  PRT214

<400>  6

Met His His His His His His His His His His Ser Ser Gly Ser Ser
1               5                   10                  15

Leu Glu Val Leu Phe Gln Gly Pro Ala Gly Ser Ser Ala Ala Ala Ala
            20                  25                  30

Ala Ala Ala Ser Gly Ser Gly Gly Tyr Gly Pro Glu Asn Gln Gly Pro
        35                  40                  45

Ser Gly Pro Val Ala Tyr Gly Pro Gly Gly Pro Val Ser Ser Ala Ala

50                  55                  60

Ala Ala Ala Ala Ala Gly Ser Gly Pro Gly Gly Tyr Gly Pro Glu Asn
65                  70                  75                  80

Gln Gly Pro Ser Gly Pro Gly Gly Tyr Gly Pro Gly Gly Ser Gly Ser
                85                  90                  95

Ser Ala Ala Ala Ala Ala Ala Ala Ala Ser Gly Pro Gly Gly Tyr Gly
            100                 105                 110

Pro Gly Ser Gln Gly Pro Ser Gly Pro Gly Gly Ser Gly Gly Tyr Gly
        115                 120                 125

Pro Gly Ser Gln Gly Ala Ser Gly Pro Gly Gly Pro Gly Ala Ser Ala
    130                 135                 140

Ala Ala Ala Ala Ala Ala Ala Ala Ala Ser Gly Pro Gly Gly Tyr Gly
145                 150                 155                 160

Pro Gly Ser Gln Gly Pro Ser Gly Pro Gly Ala Tyr Gly Pro Gly Gly
                165                 170                 175

Pro Gly Ser Ser Ala Ala Ala Ala Ala Ala Ala Ala Ser Gly Pro Gly
            180                 185                 190

Gly Tyr Gly Pro Gly Ser Gln Gly Pro Ser Gly Pro Gly Val Tyr Gly
        195                 200                 205

Pro Gly Gly Pro Gly Ser Ser Ala Ala Ala Ala Ala Ala Ala Gly Ser
    210                 215                 220

Gly Pro Gly Gly Tyr Gly Pro Glu Asn Gln Gly Pro Ser Gly Pro Gly
225                 230                 235                 240

Gly Tyr Gly Pro Gly Gly Ser Gly Ser Ser Ala Ala Ala Ala Ala Ala
                245                 250                 255

Ala Ala Ser Gly Pro Gly Gly Tyr Gly Pro Gly Ser Gln Gly Pro Ser
            260                 265                 270

Gly Pro Gly Gly Ser Gly Gly Tyr Gly Pro Gly Ser Gln Gly Gly Ser
        275                 280                 285

Gly Pro Gly Ala Ser Ala Ala Ala Ala Ala Ala Ala Ala Ser Gly Pro
    290                 295                 300

Gly Gly Tyr Gly Pro Gly Ser Gln Gly Pro Ser Gly Pro Gly Tyr Gln
305                 310                 315                 320

Gly Pro Ser Gly Pro Gly Ala Tyr Gly Pro Ser Pro Ser Ala Ser Ala
                325                 330                 335

Ser Val Ala Ala Ser Val Tyr Leu Arg Leu Gln Pro Arg Leu Glu Val
            340                 345                 350

Ser Ser Ala Val Ser Ser Leu Val Ser Ser Gly Pro Thr Asn Gly Ala
        355                 360                 365

Ala Val Ser Gly Ala Leu Asn Ser Leu Val Ser Gln Ile Ser Ala Ser
    370                 375                 380

Asn Pro Gly Leu Ser Gly Cys Asp Ala Leu Val Gln Ala Leu Leu Glu
385                 390                 395                 400

Leu Val Ser Ala Leu Val Ala Ile Leu Ser Ser Ala Ser Ile Gly Gln
                405                 410                 415

Val Asn Val Ser Ser Val Ser Gln Ser Thr Gln Met Ile Ser Gln Ala
            420                 425                 430

Leu Ser

<210>  7
<211>  355
<212>  PRT
<213>  Artificial

<220>
<223>  PRT347

<400>  7
   Met His His His His His His Ser Ser Gly Ser Ser Leu Glu Val Leu
   1               5                   10                  15
   Phe Gln Gly Pro Ala Arg Ala Gly Pro Gly Gly Tyr Arg Pro Gly Gln
               20                  25                  30
   Gln Gly Pro Ser Gly Pro Gly Ser Ala Ala Ala Ala Ala Ala Ala Ala
           35                  40                  45
   Ala Gly Pro Gly Gly Tyr Gly Pro Gly Gln Gln Gly Pro Gly Gly Tyr
       50                  55                  60
   Gly Pro Gly Gln Gln Gly Pro Ser Gly Ala Gly Ser Ala Ala Ala Ala
   65                  70                  75                  80
   Ala Ala Ala Gly Pro Gly Gln Gln Gly Leu Gly Gly Tyr Gly Pro Gly
                   85                  90                  95
   Gln Gln Gly Pro Gly Gly Tyr Gly Pro Gly Gln Gln Gly Pro Gly Gly
               100                 105                 110
   Tyr Gly Pro Gly Ser Ala Ser Ala Ala Ala Ala Ala Ala Gly Pro Gly
           115                 120                 125
   Gln Gln Gly Pro Gly Gly Tyr Gly Pro Gly Gln Gln Gly Pro Ser Gly
       130                 135                 140
   Pro Gly Ser Ala Ser Ala Ala Ala Ala Ala Ala Gly Pro Gly Gly Tyr
   145                 150                 155                 160
   Gly Pro Gly Gln Gln Gly Pro Gly Gly Tyr Ala Pro Gly Gln Gln Gly
                   165                 170                 175
   Pro Ser Gly Pro Gly Ser Ala Ala Ala Ala Ala Ala Ala Ala Arg Ala
               180                 185                 190
   Gly Pro Gly Gly Tyr Gly Pro Ala Gln Gln Gly Pro Ser Gly Pro Gly
           195                 200                 205
   Ile Ala Ala Ser Ala Ala Ser Ala Gly Pro Gly Gly Tyr Gly Pro Ala
       210                 215                 220
   Gln Gln Gly Pro Ala Gly Tyr Gly Pro Gly Ser Ala Val Ala Ala Ser
   225                 230                 235                 240
   Ala Gly Ala Gly Ser Ala Gly Tyr Gly Pro Gly Ser Gln Ala Ser Ala
                   245                 250                 255
   Ala Ala Ser Arg Leu Ala Ser Pro Asp Ser Gly Ala Arg Val Ala Ser
               260                 265                 270
   Ala Val Ser Asn Leu Val Ser Ser Gly Pro Thr Ser Ser Ala Ala Leu
           275                 280                 285
   Ser Ser Val Ile Ser Asn Ala Val Ser Gln Ile Gly Ala Ser Asn Pro
       290                 295                 300
   Gly Leu Ser Gly Cys Asp Val Leu Ile Gln Ala Leu Leu Glu Ile Val
   305                 310                 315                 320
   Ser Ala Cys Val Thr Ile Leu Ser Ser Ser Ser Ile Gly Gln Val Asn
                   325                 330                 335
   Tyr Gly Ala Ala Ser Gln Phe Ala Gln Val Val Gly Gln Ser Val Leu
               340                 345                 350
   Ser Ala Phe
           355

<210>  8
<211>  559
<212>  PRT
<213>  Artificial

<220>
<223>  PRT215

<400>  8

Met His His His His His His His His His His Ser Ser Gly Ser Ser
1               5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
        35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
        115                 120                 125

Ala Gly Gly Pro Tyr Gly Pro Gly Gly Ala Gly Gly Pro Tyr Gly Pro
    130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly

165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Gly Pro Gly
        195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
                245                 250                 255

Pro Gly Gly Tyr Gly Pro Gly Gly Ser Gly Pro Gly Gly Ser Gly Pro
            260                 265                 270

Gly Gly Tyr Gly Pro Gly Gly Ser Gly Pro Gly Gly Ser Gly Pro Gly
        275                 280                 285

Gly Tyr Gly Pro Gly Gly Ser Gly Pro Gly Gly Tyr Gly Pro Gly Gly
    290                 295                 300

Ser Gly Pro Gly Gly Ser Gly Pro Gly Gly Tyr Gly Pro Gly Gly Ser
305                 310                 315                 320

Gly Pro Gly Gly Ser Gly Pro Gly Gly Tyr Gly Pro Gly Gly Ser Gly
                325                 330                 335

Pro Gly Gly Phe Gly Pro Gly Gly Phe Gly Pro Gly Gly Ser Gly Pro
            340                 345                 350

Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
    370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
        435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
            500                 505                 510

Asn Asn Val Asn Val Leu Met Asp Ala Leu Leu Ala Ala Leu His Cys
        515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555

<210>  9
<211>  590
<212>  PRT
<213>  Artificial Sequence

<220>
<223>  Met-PRT410

<400>  9
   Met Gly Pro Gly Gln Gln Gly Pro Tyr Gly Pro Gly Ala Ser Ala Ala
   1               5                   10                  15
   Ala Ala Ala Gly Gln Asn Gly Pro Gly Ser Gly Gln Gln Gly Pro Gly
               20                  25                  30
   Gln Ser Gly Gln Tyr Gly Pro Gly Gln Gln Gly Pro Gly Gln Gln Gly
           35                  40                  45
   Pro Gly Ser Ser Ala Ala Ala Ala Ala Gly Pro Gly Gln Tyr Gly Pro
       50                  55                  60
   Gly Gln Gln Gly Pro Ser Ala Ser Ala Ala Ala Ala Ala Gly Pro Gly
   65                  70                  75                  80
   Ser Gly Gln Gln Gly Pro Gly Ala Ser Gly Gln Tyr Gly Pro Gly Gln
                   85                  90                  95
   Gln Gly Pro Gly Gln Gln Gly Pro Gly Ser Ser Ala Ala Ala Ala Ala
               100                 105                 110
   Gly Gln Tyr Gly Ser Gly Pro Gly Gln Gln Gly Pro Tyr Gly Ser Ala
           115                 120                 125
   Ala Ala Ala Ala Gly Pro Gly Ser Gly Gln Tyr Gly Gln Gly Pro Tyr
       130                 135                 140
   Gly Pro Gly Ala Ser Gly Pro Gly Gln Tyr Gly Pro Gly Gln Gln Gly
   145                 150                 155                 160
   Pro Ser Ala Ser Ala Ala Ala Ala Ala Gly Ser Gly Gln Gln Gly Pro
                   165                 170                 175
   Gly Gln Tyr Gly Pro Tyr Ala Ser Ala Ala Ala Ala Ala Gly Gln Tyr
               180                 185                 190
   Gly Ser Gly Pro Gly Gln Gln Gly Pro Tyr Gly Pro Gly Gln Ser Gly
           195                 200                 205
   Ser Gly Gln Gln Gly Pro Gly Gln Gln Gly Pro Tyr Ala Ser Ala Ala
       210                 215                 220
   Ala Ala Ala Gly Pro Gly Gln Gln Gly Pro Tyr Gly Pro Gly Ser Ser
   225                 230                 235                 240
   Ala Ala Ala Ala Ala Gly Gln Tyr Gly Tyr Gly Pro Gly Gln Gln Gly
                   245                 250                 255
   Pro Tyr Gly Pro Gly Ala Ser Gly Gln Asn Gly Pro Gly Ser Gly Gln
               260                 265                 270
   Tyr Gly Pro Gly Gln Gln Gly Pro Gly Gln Ser Ala Ala Ala Ala Ala
           275                 280                 285
   Gly Pro Gly Gln Gln Gly Pro Tyr Gly Pro Gly Ala Ser Ala Ala Ala
       290                 295                 300
   Ala Ala Gly Gln Tyr Gly Pro Gly Gln Gln Gly Pro Gly Gln Tyr Gly
   305                 310                 315                 320
   Pro Gly Ser Ser Gly Pro Gly Gln Gln Gly Pro Tyr Gly Pro Gly Ser
                       325                 330                 335
   Ser Ala Ala Ala Ala Ala Gly Gln Tyr Gly Pro Gly Gln Gln Gly Pro
                   340                 345                 350
   Tyr Gly Pro Gly Gln Ser Ala Ala Ala Ala Ala Gly Gln Tyr Gln Gln
               355                 360                 365
   Gly Pro Gly Gln Gln Gly Pro Tyr Gly Pro Gly Ala Ser Gly Pro Gly
       370                 375                 380
   Gln Gln Gly Pro Tyr Gly Pro Gly Ala Ser Ala Ala Ala Ala Ala Gly
   385                 390                 395                 400
   Pro Gly Gln Tyr Gly Pro Gly Gln Gln Gly Pro Ser Ala Ser Ala Ala
                   405                 410                 415
   Ala Ala Ala Gly Gln Tyr Gly Ser Gly Pro Gly Gln Tyr Gly Pro Tyr
               420                 425                 430
   Gly Pro Gly Gln Ser Gly Pro Gly Ser Gly Gln Gln Gly Gln Gly Pro
           435                 440                 445
   Tyr Gly Pro Gly Ala Ser Ala Ala Ala Ala Ala Gly Gln Tyr Gly Pro
       450                 455                 460
   Gly Gln Gln Gly Pro Tyr Gly Pro Gly Gln Ser Ala Ala Ala Ala Ala
   465                 470                 475                 480
   Gly Pro Gly Ser Gly Gln Tyr Gly Pro Gly Ala Ser Gly Gln Asn Gly
                   485                 490                 495
   Pro Gly Ser Gly Gln Tyr Gly Pro Gly Gln Gln Gly Pro Gly Gln Ser
               500                 505                 510
   Ala Ala Ala Ala Ala Gly Gln Tyr Gln Gln Gly Pro Gly Gln Gln Gly
           515                 520                 525
   Pro Tyr Gly Pro Gly Ala Ser Ala Ala Ala Ala Ala Gly Gln Tyr Gly
       530                 535                 540
   Ser Gly Pro Gly Gln Gln Gly Pro Tyr Gly Pro Gly Gln Ser Gly Ser
   545                 550                 555                 560
   Gly Gln Gln Gly Pro Gly Gln Gln Gly Pro Tyr Ala Ser Ala Ala Ala
                   565                 570                 575
   Ala Ala Gly Pro Gly Ser Gly Gln Gln Gly Pro Gly Ala Ser
               580                 585                 590

<210>  10
<211>  601
<212>  PRT
<213>  Artificial Sequence

<220>
<223>  PRT410

<400>  10
   Met His His His His His His Ser Ser Gly Ser Ser Gly Pro Gly Gln
   1               5                   10                  15
   Gln Gly Pro Tyr Gly Pro Gly Ala Ser Ala Ala Ala Ala Ala Gly Gln
               20                  25                  30
   Asn Gly Pro Gly Ser Gly Gln Gln Gly Pro Gly Gln Ser Gly Gln Tyr
           35                  40                  45
   Gly Pro Gly Gln Gln Gly Pro Gly Gln Gln Gly Pro Gly Ser Ser Ala
       50                  55                  60
   Ala Ala Ala Ala Gly Pro Gly Gln Tyr Gly Pro Gly Gln Gln Gly Pro
   65                  70                  75                  80
   Ser Ala Ser Ala Ala Ala Ala Ala Gly Pro Gly Ser Gly Gln Gln Gly
                   85                  90                  95
   Pro Gly Ala Ser Gly Gln Tyr Gly Pro Gly Gln Gln Gly Pro Gly Gln
               100                 105                 110
   Gln Gly Pro Gly Ser Ser Ala Ala Ala Ala Ala Gly Gln Tyr Gly Ser
           115                 120                 125
   Gly Pro Gly Gln Gln Gly Pro Tyr Gly Ser Ala Ala Ala Ala Ala Gly
       130                 135                 140
   Pro Gly Ser Gly Gln Tyr Gly Gln Gly Pro Tyr Gly Pro Gly Ala Ser
   145                 150                 155                 160
   Gly Pro Gly Gln Tyr Gly Pro Gly Gln Gln Gly Pro Ser Ala Ser Ala
                   165                 170                 175
   Ala Ala Ala Ala Gly Ser Gly Gln Gln Gly Pro Gly Gln Tyr Gly Pro
               180                 185                 190
   Tyr Ala Ser Ala Ala Ala Ala Ala Gly Gln Tyr Gly Ser Gly Pro Gly
           195                 200                 205
   Gln Gln Gly Pro Tyr Gly Pro Gly Gln Ser Gly Ser Gly Gln Gln Gly
       210                 215                 220
   Pro Gly Gln Gln Gly Pro Tyr Ala Ser Ala Ala Ala Ala Ala Gly Pro
   225                 230                 235                 240
   Gly Gln Gln Gly Pro Tyr Gly Pro Gly Ser Ser Ala Ala Ala Ala Ala
                   245                 250                 255
   Gly Gln Tyr Gly Tyr Gly Pro Gly Gln Gln Gly Pro Tyr Gly Pro Gly
               260                 265                 270
   Ala Ser Gly Gln Asn Gly Pro Gly Ser Gly Gln Tyr Gly Pro Gly Gln
           275                 280                 285
   Gln Gly Pro Gly Gln Ser Ala Ala Ala Ala Ala Gly Pro Gly Gln Gln
       290                 295                 300
   Gly Pro Tyr Gly Pro Gly Ala Ser Ala Ala Ala Ala Ala Gly Gln Tyr
   305                 310                 315                 320
   Gly Pro Gly Gln Gln Gly Pro Gly Gln Tyr Gly Pro Gly Ser Ser Gly
                   325                 330                 335
   Pro Gly Gln Gln Gly Pro Tyr Gly Pro Gly Ser Ser Ala Ala Ala Ala
               340                 345                 350
   Ala Gly Gln Tyr Gly Pro Gly Gln Gln Gly Pro Tyr Gly Pro Gly Gln
           355                 360                 365
   Ser Ala Ala Ala Ala Ala Gly Gln Tyr Gln Gln Gly Pro Gly Gln Gln
       370                 375                 380
   Gly Pro Tyr Gly Pro Gly Ala Ser Gly Pro Gly Gln Gln Gly Pro Tyr
   385                 390                 395                 400
   Gly Pro Gly Ala Ser Ala Ala Ala Ala Ala Gly Pro Gly Gln Tyr Gly
                   405                 410                 415
   Pro Gly Gln Gln Gly Pro Ser Ala Ser Ala Ala Ala Ala Ala Gly Gln
               420                 425                 430
   Tyr Gly Ser Gly Pro Gly Gln Tyr Gly Pro Tyr Gly Pro Gly Gln Ser
           435                 440                 445
   Gly Pro Gly Ser Gly Gln Gln Gly Gln Gly Pro Tyr Gly Pro Gly Ala
       450                 455                 460
   Ser Ala Ala Ala Ala Ala Gly Gln Tyr Gly Pro Gly Gln Gln Gly Pro
   465                 470                 475                 480
   Tyr Gly Pro Gly Gln Ser Ala Ala Ala Ala Ala Gly Pro Gly Ser Gly
                   485                 490                 495
   Gln Tyr Gly Pro Gly Ala Ser Gly Gln Asn Gly Pro Gly Ser Gly Gln
               500                 505                 510
   Tyr Gly Pro Gly Gln Gln Gly Pro Gly Gln Ser Ala Ala Ala Ala Ala
           515                 520                 525
   Gly Gln Tyr Gln Gln Gly Pro Gly Gln Gln Gly Pro Tyr Gly Pro Gly
       530                 535                 540
   Ala Ser Ala Ala Ala Ala Ala Gly Gln Tyr Gly Ser Gly Pro Gly Gln
   545                 550                 555                 560
   Gln Gly Pro Tyr Gly Pro Gly Gln Ser Gly Ser Gly Gln Gln Gly Pro
                   565                 570                 575
   Gly Gln Gln Gly Pro Tyr Ala Ser Ala Ala Ala Ala Ala Gly Pro Gly
               580                 585                 590
   Ser Gly Gln Gln Gly Pro Gly Ala Ser
           595                 600

<210>  11
<211>  12
<212>  PRT
<213>  Artificial Sequence

<220>
<223>  HisTag

<400>  11

Met His His His His His His Ser Ser Gly Ser Ser
1               5                   10

SEQUENCE LISTING

<110>  Spiber Inc.

<120>  METHOD FOR RECOVERING PROTEIN

<130>  MEH/92319EP1

<140>  EP17889372.3
<141>  2017-12-25

<150>  JP20160254005
<151>  2016-12-27

<160>  11

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
        35                  40                  45

Leu Ala
    50

<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210>  3
<211>  21
<212>  PRT
<213>  Araneus diadematus

<400>  3

Ser Gly Cys Asp Val Leu Val Gln Ala Leu Leu Glu Val Val Ser Ala
1               5                   10                  15

Leu Val Ser Ile Leu
            20

<210>  4
<211>  24
<212>  PRT
<213>  Artificial Sequence

<220>
<223>  His tag and start codon

<400>  4

Met His His His His His His His His His His Ser Ser Gly Ser Ser
1               5                   10                  15

Leu Glu Val Leu Phe Gln Gly Pro
            20

<210>  5
<211>  542
<212>  PRT
<213>  Artificial Sequence

<220>
<223>  PRT204

<400>  5

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala
    530                 535                 540

<210>  6
<211>  434
<212>  PRT
<213>  Artificial

<220>
<223>  PRT214

<400>  6
   Met His His His His His His His His His His Ser Ser Gly Ser Ser
   1               5                   10                  15
   Leu Glu Val Leu Phe Gln Gly Pro Ala Gly Ser Ser Ala Ala Ala Ala
               20                  25                  30
   Ala Ala Ala Ser Gly Ser Gly Gly Tyr Gly Pro Glu Asn Gln Gly Pro
           35                  40                  45
   Ser Gly Pro Val Ala Tyr Gly Pro Gly Gly Pro Val Ser Ser Ala Ala
       50                  55                  60
   Ala Ala Ala Ala Ala Gly Ser Gly Pro Gly Gly Tyr Gly Pro Glu Asn
   65                  70                  75                  80
   Gln Gly Pro Ser Gly Pro Gly Gly Tyr Gly Pro Gly Gly Ser Gly Ser
                   85                  90                  95
   Ser Ala Ala Ala Ala Ala Ala Ala Ala Ser Gly Pro Gly Gly Tyr Gly
               100                 105                 110
   Pro Gly Ser Gln Gly Pro Ser Gly Pro Gly Gly Ser Gly Gly Tyr Gly
           115                 120                 125
   Pro Gly Ser Gln Gly Ala Ser Gly Pro Gly Gly Pro Gly Ala Ser Ala
       130                 135                 140
   Ala Ala Ala Ala Ala Ala Ala Ala Ala Ser Gly Pro Gly Gly Tyr Gly
   145                 150                 155                 160
   Pro Gly Ser Gln Gly Pro Ser Gly Pro Gly Ala Tyr Gly Pro Gly Gly
                   165                 170                 175
   Pro Gly Ser Ser Ala Ala Ala Ala Ala Ala Ala Ala Ser Gly Pro Gly
               180                 185                 190
   Gly Tyr Gly Pro Gly Ser Gln Gly Pro Ser Gly Pro Gly Val Tyr Gly
           195                 200                 205
   Pro Gly Gly Pro Gly Ser Ser Ala Ala Ala Ala Ala Ala Ala Gly Ser
       210                 215                 220
   Gly Pro Gly Gly Tyr Gly Pro Glu Asn Gln Gly Pro Ser Gly Pro Gly
   225                 230                 235                 240
   Gly Tyr Gly Pro Gly Gly Ser Gly Ser Ser Ala Ala Ala Ala Ala Ala
                   245                 250                 255
   Ala Ala Ser Gly Pro Gly Gly Tyr Gly Pro Gly Ser Gln Gly Pro Ser
               260                 265                 270
   Gly Pro Gly Gly Ser Gly Gly Tyr Gly Pro Gly Ser Gln Gly Gly Ser
           275                 280                 285
   Gly Pro Gly Ala Ser Ala Ala Ala Ala Ala Ala Ala Ala Ser Gly Pro
       290                 295                 300
   Gly Gly Tyr Gly Pro Gly Ser Gln Gly Pro Ser Gly Pro Gly Tyr Gln
   305                 310                 315                 320
   Gly Pro Ser Gly Pro Gly Ala Tyr Gly Pro Ser Pro Ser Ala Ser Ala
                   325                 330                 335
   Ser Val Ala Ala Ser Val Tyr Leu Arg Leu Gln Pro Arg Leu Glu Val
               340                 345                 350
   Ser Ser Ala Val Ser Ser Leu Val Ser Ser Gly Pro Thr Asn Gly Ala
           355                 360                 365
   Ala Val Ser Gly Ala Leu Asn Ser Leu Val Ser Gln Ile Ser Ala Ser
       370                 375                 380
   Asn Pro Gly Leu Ser Gly Cys Asp Ala Leu Val Gln Ala Leu Leu Glu
   385                 390                 395                 400
   Leu Val Ser Ala Leu Val Ala Ile Leu Ser Ser Ala Ser Ile Gly Gln
                   405                 410                 415
   Val Asn Val Ser Ser Val Ser Gln Ser Thr Gln Met Ile Ser Gln Ala
               420                 425                 430
   Leu Ser

<210>  7
<211>  355
<212>  PRT
<213>  Artificial

<220>
<223>  PRT347

<400>  7

Met His His His His His His Ser Ser Gly Ser Ser Leu Glu Val Leu
1               5                   10                  15

Phe Gln Gly Pro Ala Arg Ala Gly Pro Gly Gly Tyr Arg Pro Gly Gln
            20                  25                  30

Gln Gly Pro Ser Gly Pro Gly Ser Ala Ala Ala Ala Ala Ala Ala Ala
        35                  40                  45

Ala Gly Pro Gly Gly Tyr Gly Pro Gly Gln Gln Gly Pro Gly Gly Tyr
    50                  55                  60

Gly Pro Gly Gln Gln Gly Pro Ser Gly Ala Gly Ser Ala Ala Ala Ala
65                  70                  75                  80

Ala Ala Ala Gly Pro Gly Gln Gln Gly Leu Gly Gly Tyr Gly Pro Gly
                85                  90                  95

Gln Gln Gly Pro Gly Gly Tyr Gly Pro Gly Gln Gln Gly Pro Gly Gly
            100                 105                 110

Tyr Gly Pro Gly Ser Ala Ser Ala Ala Ala Ala Ala Ala Gly Pro Gly
        115                 120                 125

Gln Gln Gly Pro Gly Gly Tyr Gly Pro Gly Gln Gln Gly Pro Ser Gly
    130                 135                 140

Pro Gly Ser Ala Ser Ala Ala Ala Ala Ala Ala Gly Pro Gly Gly Tyr
145                 150                 155                 160

Gly Pro Gly Gln Gln Gly Pro Gly Gly Tyr Ala Pro Gly Gln Gln Gly
                165                 170                 175

Pro Ser Gly Pro Gly Ser Ala Ala Ala Ala Ala Ala Ala Ala Arg Ala
            180                 185                 190

Gly Pro Gly Gly Tyr Gly Pro Ala Gln Gln Gly Pro Ser Gly Pro Gly
        195                 200                 205

Ile Ala Ala Ser Ala Ala Ser Ala Gly Pro Gly Gly Tyr Gly Pro Ala
    210                 215                 220

Gln Gln Gly Pro Ala Gly Tyr Gly Pro Gly Ser Ala Val Ala Ala Ser
225                 230                 235                 240

Ala Gly Ala Gly Ser Ala Gly Tyr Gly Pro Gly Ser Gln Ala Ser Ala
                245                 250                 255

Ala Ala Ser Arg Leu Ala Ser Pro Asp Ser Gly Ala Arg Val Ala Ser
            260                 265                 270

Ala Val Ser Asn Leu Val Ser Ser Gly Pro Thr Ser Ser Ala Ala Leu
        275                 280                 285

Ser Ser Val Ile Ser Asn Ala Val Ser Gln Ile Gly Ala Ser Asn Pro
    290                 295                 300

Gly Leu Ser Gly Cys Asp Val Leu Ile Gln Ala Leu Leu Glu Ile Val
305                 310                 315                 320

Ser Ala Cys Val Thr Ile Leu Ser Ser Ser Ser Ile Gly Gln Val Asn
                325                 330                 335

Tyr Gly Ala Ala Ser Gln Phe Ala Gln Val Val Gly Gln Ser Val Leu
            340                 345                 350

Ser Ala Phe
        355

<210>  8
<211>  559
<212>  PRT
<213>  Artificial

<220>
<223>  PRT215

<400>  8

Met His His His His His His His His His His Ser Ser Gly Ser Ser
1               5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
        35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
        115                 120                 125

Ala Gly Gly Pro Tyr Gly Pro Gly Gly Ala Gly Gly Pro Tyr Gly Pro
    130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly
                165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Gly Pro Gly
        195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
                245                 250                 255

Pro Gly Gly Tyr Gly Pro Gly Gly Ser Gly Pro Gly Gly Ser Gly Pro
            260                 265                 270

Gly Gly Tyr Gly Pro Gly Gly Ser Gly Pro Gly Gly Ser Gly Pro Gly
        275                 280                 285

Gly Tyr Gly Pro Gly Gly Ser Gly Pro Gly Gly Tyr Gly Pro Gly Gly
    290                 295                 300

Ser Gly Pro Gly Gly Ser Gly Pro Gly Gly Tyr Gly Pro Gly Gly Ser
305                 310                 315                 320

Gly Pro Gly Gly Ser Gly Pro Gly Gly Tyr Gly Pro Gly Gly Ser Gly
                325                 330                 335

Pro Gly Gly Phe Gly Pro Gly Gly Phe Gly Pro Gly Gly Ser Gly Pro
            340                 345                 350

Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
    370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
        435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
            500                 505                 510

Asn Asn Val Asn Val Leu Met Asp Ala Leu Leu Ala Ala Leu His Cys
        515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555

<210>  9
<211>  590
<212>  PRT
<213>  Artificial Sequence

<220>
<223>  Met-PRT410

<400>  9

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser

545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  10
<211>  601
<212>  PRT
<213>  Artificial Sequence

<220>
<223>  PRT410

<400>  10

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln

420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  11
<211>  12
<212>  PRT
<213>  Artificial Sequence

<220>
<223>  HisTag

<400>  11

Met His His His His His His Ser Ser Gly Ser Ser
1               5                   10

## Description

### Technical Field

The present invention relates to a method for recovering protein.

### Background Art

A method of recycling (recovering) a material from waste is very useful from a point of environmental protection. Under such a circumstance, various developments have been made on a method of recycling a specific material from waste.

For example, Patent Literature 1 discloses a chemical recycling method of polyethylene terephthalate waste.

### Citation List

### Patent Literature

[Patent Literature 1] Japanese Patent No. 3715812

### Summary of Invention

### Problems to be Solved by the Invention

Here, structural protein that is excellent in terms of strength and the like is a material useful as a substitute for a petroleum-derived material. However, recycling from waste is mainly related to the petroleum-derived material, and no method has been known that can recover a target protein from waste containing protein such as structural protein.

Therefore, an object of the present invention is to provide a method that can recover a target protein from a mixture containing the target protein and a material different from the target protein.

### Means for Solving the Problems

The present invention provides a method for recovering protein, which recovers a target protein from a mixture containing the target protein and a material different from the target protein, the method including a dissolution step of dissolving either the target protein or the material by applying pressure while heating to a solution for dissolution containing the mixture and a polar solvent, and a separation step of separating an obtained solution.

According to the present invention, since either the target protein or the material is dissolved by applying pressure while heating to the solution for dissolution containing the mixture and the polar solvent, for example, the target protein can be recovered from the mixture, by using solid-liquid separation.

The dissolution step is preferably a step of dissolving the target protein. Accordingly, it is possible to recover the target protein with even higher purity.

The target protein may be one or more proteins selected from the group consisting of silk fibroin, spider silk fibroin, and hornet silk fibroin.

The polar solvent preferably includes one or more solvents selected from the group consisting of water, alcohol, dimethyl sulfoxide, dimethylformamide, and hexafluoroacetone. Accordingly, it becomes easier to recover the target protein from the mixture.

The material may include one or more materials selected from the group consisting of polyester, nylon, cotton, and wool.

### Effects of the Invention

According to the present invention, it is possible to recover a target protein from a mixture containing the target protein and a material different from the target protein.

### Brief Description of Drawings

FIG. 1 is a graph showing analysis results of GPC in Example 1.
FIG. 2 is a graph showing analysis results of GPC in Example 5.

### Description of Embodiments

Hereinafter, embodiments of the present invention will be described in detail. However, the present invention is not limited to the following embodiments.

A method according to the present embodiment is a method for recovering a target protein from a mixture containing the target protein and a material different from the target protein, the method including a dissolution step of dissolving either the target protein or the material by applying pressure while heating to a solution for dissolution containing the mixture and a polar solvent, and a separation step of separating an obtained solution.

When applying the pressure while heating to the solution for dissolution containing the mixture and the polar solvent, it is possible to improve solubility of either the target protein or the material different from the target protein. Accordingly, it is possible to recover the target protein from the mixture.

The dissolution step is preferably a step of dissolving the target protein. When dissolving the target protein in the solution for dissolution, it is possible to recover the target protein with higher purity.

In a case where the dissolution step is the step of dissolving the target protein, the entire amount of the target protein does not have to be dissolved, as long as a part of the protein may be dissolved.

Also, the polar solvent contained in the solution for dissolution may include, for example, one or more solvents selected from the group consisting of water, alcohol, dimethyl sulfoxide (DMSO), dimethylformamide (DMF), and hexafluoroacetone (HFA). From a viewpoint of recovering the target protein more efficiently, the polar solvent can be water alone or a mixed solvent of alcohol and water. From a viewpoint of reducing a negative impact on the environment, the polar solvent can be water. From a viewpoint of obtaining the target protein under a milder condition, the polar solvent may also contain alcohol. For example, the polar solvent may also be a mixed solvent of water and alcohol, a mixed solvent of dimethyl sulfoxide and alcohol, or a mixed solvent of water, alcohol, and dimethyl sulfoxide. A proportion of the alcohol relative to the total amount of the polar solvent (or the mixed solvent) may be 5% to 100% by mass, or 10% to 50% by mass. When the polar solvent containing alcohol is used, there is a tendency that the target protein can be dissolved in the polar solvent at a lower pressure.

In the present specification, the "alcohol" means a compound including an aliphatic group which may have a substituent and a hydroxyl group bonded to the aliphatic group. The aliphatic group may be substituted with, for example, a halogen atom such as a fluorine atom or may be unsubstituted. Examples of a fluoroalcohol having an aliphatic group substituted with a fluorine atom include hexafluoroisopropanol (HFIP).

Alcohol having a low boiling point is particularly advantageous in a point that conditions such as preparation of an alcohol solution, a concentration thereof, and formation of a formed product can be made mild. From such a viewpoint, the boiling point of the alcohol may be, for example, 99°C or lower and 50°C or higher at 1 atm. The boiling point of the alcohol may be 60°C or higher at 1 atm. In addition, in general, alcohol having one hydroxyl group tends to have a lower boiling point than that of alcohol having two or more hydroxyl groups.

The carbon number of the alcohol (the carbon number of the aliphatic group) is not particularly limited, and may be 1 to 10. In particular, from the viewpoint that the target protein can be recovered under a mild condition, the carbon numbers of the alcohol may be 2 to 8 or 2 to 5. The alcohol contained in the polar solvent may be, for example, one or more alcohols having 1 to 10 carbon atoms, selected from the group consisting of methanol, ethanol, propanol, butanol, pentanol, hexanol, heptanol, octanol, nonanol, decanol, and isomers of these alcohols, one or more alcohols having 2 to 5 carbon atoms, selected from the group consisting of ethanol, propanol, butanol, and isomers of these alcohols, and one or more alcohols selected from the group consisting of ethanol, 1-propanol, and 2-propanol.

The target protein may also be a structural protein. The structural protein means protein which forms or maintains a structure, a form, and the like, in vivo. The structural protein includes hydrophobic protein and a polypeptide prone to self-aggregation in a polar solvent. Since these structural proteins generally have low solubility to a polar solvent, the method of the present embodiment is particularly useful for recovering these structural proteins.

The target protein may also be fibroin. The fibroin may be, for example, one or more selected from the group consisting of silk fibroin, spider silk fibroin, and hornet silk fibroin. The target protein may be silk fibroin, spider silk fibroin, or a combination thereof. In a case where the silk fibroin and the spider silk fibroin are used in combination, a proportion of the silk fibroin may be, for example, 40 parts by mass or less, 30 parts by mass or less, or 10 parts by mass or less, relative to 100 parts by mass of the spider silk fibroin.

Silk is a fiber obtained from a cocoon made by a silkworm that is a larva of a silkworm moth (Bombyx mori). In general, one silk thread is formed of two silk fibroin and a shell material (sericin) covering these from outside. The silk fibroin is formed of a number of fibrils. The silk fibroin is covered with four layers of the sericin. In practical, a silk filament obtained by dissolving and removing the sericin on an outer side by refining is used for a clothing application. Common silk has a specific gravity of 1.33, a fineness of 3.3 decitex on an average, and a fiber length of approximately 1300 to 1500 m. The silk fibroin is obtained by using natural or domestic silkworm cocoons or used or discarded silk fabrics, as a raw material.

The silk fibroin may be sericin-removed silk fibroin, sericin-unremoved silk fibroin, or a combination thereof. The sericin-removed silk fibroin is a powder obtained by lyophilizing silk fibroin purified by removing the sericin covering the silk fibroin and other fats and the like. The sericin-unremoved silk fibroin is unpurified fibroin from which the sericin and the like are not removed.

The spider silk fibroin may contain a spider silk polypeptide selected from the group consisting of natural spider silk protein and a polypeptide derived from the natural spider silk protein.

Examples of the natural spider silk protein include large spinneret tube dragline silk protein, weft silk protein, and minor ampullate gland protein. It is presumed that since the large spinneret tube dragline silk has a repetitive region including a crystalline region and an amorphous region, it has both high stress and stretchability. It is a major feature that the weft of spider silk has a repetitive region including an amorphous region without a crystalline region. On the other hand, the weft is inferior in stress as compared to the large spinneret tube dragline silk, but has high stretchability. It is considered that this is because most of the weft is formed of the amorphous region.

The large spinneret tube dragline silk protein has features that it is produced in a major ampullate gland of a spider and is excellent in toughness. Examples of the large spinneret tube dragline silk protein include the major ampullate spidroins MaSp1 and MaSp2 derived from an America silk spider (Nephila clavipes) and ADF3 and ADF4 derived from a European garden spider (Araneus diadematus). The ADF3 is one of two major dragline silk proteins of the European garden spider. A polypeptide derived from the natural spider silk protein may be a polypeptide derived from these dragline silk proteins. The polypeptide derived from the ADF3 is relatively easy to be synthesized, and has excellent properties in terms of strength and toughness.

The weft silk protein is produced in a flagelliform gland of the spider. Examples of the weft silk protein include flagelliform silk protein derived from the America silk spider (Nephila clavipes).

A polypeptide derived from the natural spider silk protein may be a recombinant spider silk protein. Examples of the recombinant spider silk protein include a mutant, an analogue, or a derivative of the natural spider silk protein. A preferable example of such a polypeptide is the recombinant spider silk protein of the large spinneret tube dragline silk protein (also referred to as "a polypeptide derived from the large spinneret tube dragline silk protein").

The polypeptide derived from the large spinneret tube dragline silk protein may include two or more, five or more, or ten or more units (also referred to as a motif) of an amino acid sequence represented by Formula 1: REP 1-REP2 (1). An upper limit of the number of the units of the amino acid sequence represented by Formula 1: REP1-REP2 (1) is not particularly limited, and may be, for example, 300 or less, or 200 or less. Alternatively, the polypeptide derived from the large spinneret tube dragline silk protein may be a polypeptide, having a unit of the amino acid sequence represented by Formula 1: REP1-REP2 (1), in which a C-terminus sequence is an amino acid sequence shown in any of SEQ ID NOs: 1 to 3 or an amino acid sequence having 90% or more of homology with the amino acid sequence shown in any of SEQ ID NOs: 1 to 3. In the polypeptide derived from the large spinneret tube dragline silk protein, the units of the amino acid sequence represented by Formula 1: REP1-REP2 (1) may be the same as or different from each other.

In Formula 1, a proportion of the number of alanine residues to the total number of the amino acid residues in REP1 motif is usually 83% or more, and may be 86% or more, 90% or more, or 95% or more. REP1 may be a polyalanine in which a ratio of the number of the alanine residues is 100%. Consecutive alanine (Ala) in a row in REP1 may have two or more residues, three or more residues, four or more residues, or five or more residues. The consecutive alanine in a row in REP1 may have 20 residues or less, 16 residues or less, 12 residues or less, or 10 residues or less. REP1 motif may also include other amino acid residues selected from serine (Ser), glycine (Gly), glutamine (Gln), and the like, in addition to alanine (Ala).

In Formula 1, REP2 is an amino acid sequence consisting of 10 to 200 amino acid residues, and the total number of the residues of glycine (Gly), serine (Ser), glutamine (Gin), and alanine (Ala) contained in the amino acid sequence may be 40% or more, 60% or more, or 70% or more relative to the total number of the amino acid residues.

In the large spinneret tube dragline silk, REP1 corresponds to a crystalline region that forms a crystalline beta sheet within a fiber, and REP2 corresponds to an amorphous region that is more flexible and lacks largely regular structure. [REP1-REP2] corresponds to a repetitive region (repetitive sequence) including the crystalline region and the amorphous region, and is a characteristic sequence of a dragline silk protein.

The amino acid sequence shown in SEQ ID NO: 1 is the same as an amino acid sequence consisting of 50 amino acid residues at the C-terminus of the amino acid sequence (NCBI accession No: AAC47010, GI: 1263287) of ADF3. The amino acid sequence shown in SEQ ID NO: 2 is the same as an amino acid sequence obtained by removing 20 residues from the C-terminus of the amino acid sequence shown in SEQ ID NO: 1. The amino acid sequence shown in SEQ ID NO: 3 is the same as an amino acid sequence obtained by removing 29 residues from the C-terminus of the amino acid sequence shown in SEQ ID NO: 1.

The polypeptide including two or more units of the amino acid sequence represented by Formula 1: REP1-REP2 (1) can be, for example, a polypeptide consisting of an amino acid sequence shown in SEQ ID NO: 5. The polypeptide consisting of the amino acid sequence shown in SEQ ID NO: 5 is obtained by mutating the amino acid sequence (NCBI accession No: AAC47010, GI: 1263287) of ADF3, in which an amino acid sequence (SEQ ID NO: 4) consisting of an initiation codon, His10 tag, and Human rhinovirus 3C protease (HRV3C protease) recognition site to an N-terminus, such that translation is terminated at a 543rd amino acid residue.

The polypeptide including two or more units of the amino acid sequence represented by Formula 1: REP1-REP2 (1) consists of an amino acid sequence in which one or more amino acids are substituted, deleted, inserted, and/or added in the amino acid sequence shown in SEQ ID NO: 5, and can be protein including a repetitive region including a crystal region and an amorphous region. In the present specification, "one or more" means, a range selected from, for example, 1 to 40, 1 to 35, 1 to 30, 1 to 25, 1 to 20, 1 to 15, 1 to 10, and 1 or several. In the present specification, "one or several" is 1 to 9, 1 to 8, 1 to 7, 1 to 6, 1 to 5, 1 to 4, 1 to 3, 1 or 2, or 1.

The polypeptide including two or more units of the amino acid sequence represented by Formula 1: REP1-REP2 (1) may be a recombinant protein derived from ADF4 having the amino acid sequence shown in SEQ ID NO: 6. The amino acid sequence shown in SEQ ID NO: 6 is obtained by adding the amino acid sequence (SEQ ID NO: 4) consisting of an initiation codon, His10 tag, and Human rhinovirus 3C protease (HRV3C protease) recognition site to the N-terminus of a partial amino acid sequence (NCBI accession No: AAC47011, GI: 1263289) of ADF4 obtained from the NCBI database.

The polypeptide including two or more units of the amino acid sequence represented by Formula 1: REP1-REP2 (1) consists of an amino acid sequence in which one or more amino acids are substituted, deleted, inserted, and/or added in the amino acid sequence shown in SEQ ID NO: 6, and can be a polypeptide including a repetitive region including a crystal region and an amorphous region.

The polypeptide including two or more units of the amino acid sequence represented by Formula 1: REP1-REP2 (1) may be a recombinant protein derived from MaSp2 having an amino acid sequence shown in SEQ ID NO: 7. The amino acid sequence shown in SEQ ID NO: 7 is obtained by adding an amino acid sequence (SEQ ID NO: 11) consisting of an initiation codon, His10 tag, and Human rhinovirus 3C protease (HRV3C protease) recognition site to an N-terminus of a partial sequence (NCBI accession No: AAT75313, GI: 50363147) of MaSp2 obtained from the NCBI database.

The polypeptide including two or more units of the amino acid sequence represented by Formula 1: REP1-REP2 (1) consists of an amino acid sequence in which one or more amino acids are substituted, deleted, inserted, and/or added in the amino acid sequence shown in SEQ ID NO: 7, and can be a polypeptide including a repetitive region including a crystal region and an amorphous region.

The polypeptide derived from the weft silk protein may include 10 or more, 20 or more, or 30 or more units of an amino acid sequence represented by Formula 2: REP3 (2). An upper limit of the number of the units of the amino acid sequence represented by Formula 2: REP3 (2) is not particularly limited, and may be, for example, 300 or less, or 200 or less.

In Formula 2, REP3 represents an amino acid sequence consisting of Gly-Pro-Gly-Gly-X, and X represents one amino acid selected from the group consisting of alanine (Ala), serine (Ser), tyrosine (Tyr), and valine (Val).

The polypeptide including 10 or more units of the amino acid sequence represented by Formula 2: REP3 (2) may be a recombinant protein derived from flagelliform silk protein having an amino acid sequence shown in SEQ ID NO: 8. The amino acid sequence shown in SEQ ID NO: 8 is an amino acid sequence obtained in a manner that an amino acid sequence (referred to as PR1 sequence) from 1220th residue to the 1659th residue from the N-terminus, corresponding to a repeat portion and a motif of the partial sequence (NCBI accession number: AAF36090, GI: 7106224) of the flagelliform silk protein of the America silk spider obtained from the NCBI database is bonded to a C-terminus amino acid sequence from 816th residue to 907th residue from the C-terminus of the partial sequence (NCBI accession number: AAC38847, GI: 2833649) of the flagelliform silk protein of the America silk spider obtained from the NCBI database, and the amino acid sequence (SEQ ID NO: 4) consisting of an initiation codon, His10 tag, and HRV3C protease recognition site is added to an N-terminus of the bonded sequence.

The polypeptide including 10 or more units of the amino acid sequence represented by Formula 2: REP3 (2) consists of an amino acid sequence in which one or more amino acids are substituted, deleted, inserted, and/or added in the amino acid sequence shown in SEQ ID NO: 8, and can be a polypeptide including a repetitive region including an amorphous region.

From a viewpoint of productivity when performing recombinant protein production using a microorganism such as E. coli as a host, a molecular weight of the protein or the polypeptide may be 500 kDa or less, 300 kDa or less, 200 kDa or less, or 100 kDa or less, and may be 10 kDa or more.

The hornet silk fibroin is protein produced by a larva or a bee and may contain a polypeptide selected from the group consisting of natural hornet silk protein and a polypeptide derived from the natural hornet silk protein.

The polypeptide can be produced, for example, using a host transformed with an expression vector containing a gene encoding the polypeptide.

A method for producing the gene encoding the polypeptide is not particularly limited. For example, in a case of a natural spider silk protein, a gene can be manufactured by a method of amplifying and cloning the gene encoding the protein by polymerase chain reaction (PCR) or the like from cells derived from a spider, or by chemical synthesis. A chemical synthesis method of the gene is not particularly limited. For example, the gene can be chemically synthesized by a method that oligonucleotides automatically synthesized by AKTA oligopilot plus 10/100 (GE Healthcare Japan Co., Ltd.) or the like are linked by PCR and the like, based on amino acid sequence information of the natural spider silk protein obtained from the NCBI web database and the like. In this case, in order to facilitate purification and confirmation of the protein, a gene encoding protein consisting of an amino acid sequence in which an amino acid sequence including initiation codon and His 10 tag is added to the N terminus of the amino acid sequence may be synthesized.

As an expression vector, a plasmid, phage, virus, or the like capable of expressing protein from a DNA sequence can be used. The plasmid expression vector is not particularly limited as long as it can express a target gene in a host cell and can amplify itself. For example, in a case where E. coli Rosetta (DE3) is used as a host, pET22b (+) plasmid vector, pCold plasmid vector, and the like can be used. Among these, from a viewpoint of productivity of protein, the pET22b (+) plasmid vector can be used. As the host, for example, an animal cell, a plant cell, and a microorganism can be used.

A combination of the target protein such as the silk fibroin and the spider silk fibroin with other proteins may be included in a solution to be obtained from the solution for dissolution and the combination. Examples of the other proteins include collagen, soy protein, casein, keratin, and whey protein. When using the other proteins in combination with the target protein, it is possible to appropriately adjust a physical property derived from the target protein. A proportion of the other proteins may be, for example, 40 parts by mass or less, 30 parts by mass or less, or 10 parts by mass or less, relative to 100 parts by mass of the target protein.

A concentration of the target protein in the solution for dissolution may be 15% by mass or more, 30% by mass or more, 40% by mass or more, or 50% by mass or more, based on the mass of the polar solvent. From a viewpoint of recovery efficiency of the target protein, the concentration of the target protein may be 70% by mass or less, 65% by mass or less, or 60% by mass or less, based on the mass of the polar solvent.

The material different from the target protein may be an inorganic material or an organic material. Examples of the inorganic material include metal, a carbon fiber, glass, or a combination thereof. Examples of the organic material include a cellulose fiber such as polyester, nylon, cotton, wool, and rayon, aramid, polytetrafluoroethylene (PTFE), polyurethane, a bioplastic fiber such as biopolyester represented by polylactic acid (PLA), bio nylon, or bioPET, or a combination thereof. Examples of the polyester include polyethylene terephthalate (PET), polyethylene naphthalate (PEN), and polytributylene terephthalate (PTT). In addition, the wool is an animal fiber of which a main component is keratin. The cotton is a vegetable fiber of which a main component is cellulose. The nylon is a hydrophobic fiber which is a type of polyamide.

The material different from the target protein may include one or more materials selected from the group consisting of polyester, nylon, cotton, and wool.

The solution for dissolution may further include one or more inorganic salts. When adding the inorganic salt to the solution for dissolution, an effect of improving the solubility by heating and pressing can be more remarkable. Examples of the inorganic salt include an inorganic salt including Lewis acid and Lewis base shown below. The Lewis base may be, for example, an oxo acid ion (such as a nitrate ion and a perchlorate ion), a metal oxo acid ion (such as a permanganate ion), a halide ion, a thiocyanate ion, and a cyanate ion. The Lewis acid may be, for example, a metal ion such as an alkali metal ion and an alkaline earth metal ion, a polyatomic ion such as an ammonium ion, and a complex ion. Specific examples of the inorganic salt include lithium salts such as lithium chloride, lithium bromide, lithium iodide, lithium nitrate, lithium perchlorate, and lithium thiocyanate, calcium salts such as calcium chloride, calcium bromide, calcium iodide, calcium nitrate, calcium perchlorate, and calcium thiocyanate, iron salts such as iron chloride, iron bromide, iron iodide, iron nitrate, iron perchlorate, and iron thiocyanate, aluminum salts such as aluminum chloride, aluminum bromide, aluminum iodide, aluminum nitrate, aluminum perchlorate, and aluminum thiocyanate, potassium salts such as potassium chloride, potassium bromide, potassium iodide, potassium nitrate, potassium perchlorate, and potassium thiocyanate, sodium salts such as sodium chloride, sodium bromide, sodium iodide, sodium nitrate, sodium perchlorate, and sodium thiocyanate, zinc salts such as zinc chloride, zinc bromide, zinc iodide, zinc nitrate, zinc perchlorate, and zinc thiocyanate, magnesium salts such as magnesium chloride, magnesium bromide, magnesium iodide, magnesium nitrate, magnesium perchlorate, and magnesium thiocyanate, barium salts such as barium chloride, barium bromide, barium iodide, barium nitrate, barium perchlorate, and barium thiocyanate, and strintium salts such as strintium chloride, strintium bromide, strintium iodide, strintium nitrate, strintium perchlorate, and strintium thiocyanate. A concentration of the inorganic salt may be 1.0% by mass or more, 5.0% by mass or more, 9.0% by mass or more, 15.0% by mass or more, or 20.0% by mass or more, based on the total mass of the target protein. The concentration of the inorganic salt may also be 30% by mass or less, 25% by mass or less, or 20% by mass or less, based on the total mass of the target protein.

The solution for dissolution can include various additives, as needed. Examples of the additive include a plasticizer, a crystal nucleating agent, an antioxidant, an ultraviolet absorber, a coloring agent, a crosslinking agent, a polymerization inhibitor, a filler, and a synthetic resin. A concentration of the additive may be 50% by mass or less, based on the total mass the target protein.

When applying a predetermined pressure while heating to the solution for dissolution, either the target protein or the material can be dissolved. Here, when applying pressure to the solution for dissolution, even at a relatively low temperature, it is possible to dissolve either the target protein or the material. Therefore, it is possible to recover the target protein while preventing denaturation, gelation, and degradation of the target protein from being caused. Further, in a case where the target protein is dissolved in the dissolution step, since a concentration step using dialysis or the like is not necessarily required, it is also possible to obtain a protein solution with high production efficiency.

The pressure applied to the solution for dissolution is adjusted such that it is possible to recover the target protein, according to types of the target protein and the polar solvent, a desired concentration, and the like. When the pressure applied to the solution for dissolution is high, the solubility tends to be higher. The pressure applied to the solution for dissolution can be 0.05 MPa or more, 0.06 MPa or more, 0.07 MPa or more, 0.08 MPa or more, 0.1 MPa or more, 1.0 MPa or more, 5.0 MPa or more, or 10 MPa or more. The pressure applied to the solution for dissolution can be 300 MPa or less, 150 MPa or less, 50 MPa or less, or 30 MPa or less.

A method of applying the pressure to the solution for dissolution is not particularly limited. For example, a method of heating a solvent and applying pressure by vapor pressure of the solvent in a pressure resistant vessel, a method of applying pressure by adjusting the pressure in a sealed pressure resistant vessel by filling the vessel with an inert gas such as nitrogen and argon or air may be applied.

The pressure may be applied to the solution for dissolution while heating the solution for dissolution. It is not limited that the heating is performed during applying the pressure. For example, the solution for dissolution is heated to a predetermined temperature and then the pressure may be applied to the solution for dissolution. From a viewpoint of further preventing the protein from degrading, a heating temperature may be 150°C or lower, 140°C or lower, 135°C or lower, or 130°C or lower. In a case where the polar solvent is water, from a viewpoint of further preventing water-mediated protein from degrading, the heating temperature is preferably 140°C or lower. From a viewpoint of further improving the solubility, the heating temperature may be 70°C or higher, 90°C or higher, or 100°C or higher. These upper and lower limits can be used in any combination. For example, the heating temperature may be 70°C to 150°C, 90°C to 140°C, or 100°C to 130°C. In addition, for example, in a case where the polar solvent is a mixed solvent of water and alcohol, the heating temperature may be 150°C or lower, 140°C or lower, 135°C or lower, or 130°C or lower from the viewpoint of further preventing the protein from degrading, and may be 70°C or higher, 80°C or higher, or 90°C or higher from the viewpoint of improving the solubility. These upper and lower limits can be used in any combination. For example, the heating temperature in the case where the polar solvent is the mixed solvent of water and alcohol may be 70°C to 150°C, 80°C to 140°C, or 90°C to 130°C. The heating temperature may be a constant temperature or may vary.

The pressure may be applied to the solution for dissolution while stirring the solution for dissolution. It is not limited that the stirring is performed during applying the pressure. The solution for dissolution may be stirred before and after the pressure is applied thereto. A stirring method is not particularly limited. For example, the solution for dissolution can be stirred using an inclined blade, a turbine blade, or the like.

The solution obtained after pressurization may include a gas for pressurization. Therefore, the method for recovering protein according to an embodiment may further include removing a gas from the solution. A method of removing the gas is not particularly limited, and examples thereof include a method using a centrifuge. When applying the solution to the centrifuge, it is possible to remove a layer including a relatively large amount of gas.

The method for recovering protein according to the present embodiment includes the separation step of separating the solution obtained in the dissolution step.

The separation step may be performed by a usual solid-liquid separation treatment. For example, the solution and insoluble matter may be separated from each other by filtration.

In a case where the dissolution step is a step of dissolving the target protein, the concentration of the target protein dissolved in the solution is 1% by mass or more, 5% by mass or more, 10% by mass or more, 15% by mass or more, 20 mass% or more, or 30 mass% or more, and 50 mass% or less, 45 mass% or less, or 40 mass% or less, based on the mass of the solution.

The method for recovering protein according to the present embodiment may include a step of washing off insoluble matter by using a polar solvent such as water, alcohol, dimethyl sulfoxide (DMSO), dimethylformamide (DMF), hexafluoroacetone (HFA), and hexafluoro-2-propanol (HFIP). As the alcohol, the alcohols described above can be used.

In the method for recovering protein according to the present embodiment, in a case where the dissolution step is a step of dissolving the target protein, for example, the target protein can be recovered from the solution, by applying a method of removing the polar solvent from the solution and a common method such as reprecipitation. In this case, any form of protein such as powder may be recovered from the solution including the target protein, or it may be used directly to produce a formed product.

In a case where the dissolution step is a step of dissolving the target protein, the solution including the target protein can be used, for example, to produce a formed product of protein by various methods. For example, a formed product containing protein may be obtained by removing the polar solvent from the solution containing the target protein. In particular, a solution containing spider silk fibroin can be used to produce a formed product having excellent physical property making the most of the properties of the spider silk fibroin.

In a case where the dissolution step is a step of dissolving the target protein, for example, a formed product such as a gel, a film, or a fiber can be produced by using the solution as a dope solution. The film can be produced, for example, by a method of forming a film of the solution (the dope solution) and removing the polar solvent from the formed film. The fiber can be produced, for example, by a method of spinning the solution and removing the polar solvent from the spun solution.

In the method for recovering protein, it can be confirmed whether the recovered protein is the target protein, for example, using gel permeation chromatography (GPC), infrared spectroscopy (IR), polyacrylamide gel electrophoresis (SDS-PAGE), mass spectrometry (MS), and nuclear magnetic resonance (NMR).

### Examples

Hereinafter, the present invention will be described in more detail, based on Examples. However, the present invention is not limited to the following Examples.

### [Preparation of Spider Silk Protein]

### (1) Synthesis of Nucleic Acid Encoding Modified Fibroin and Construction of Expression Vector

Based on a nucleotide sequence and an amino acid sequence of Nephila clavipes (GenBank accession number: P46804.1, GI: 1174415) which is a naturally derived spider silk fibroin, a modified fibroin having the amino acid sequences respectively shown in SEQ ID NOs: 9 and 10 was designed. The amino acid sequence shown in SEQ ID NO: 9 was obtained in a manner that, starting from the naturally derived fibroin, an amino acid sequence in which the alanine residues are continuous in the (A)n motif was deleted such that the number of the consecutive alanine residues was 5, every two (A)n motif ((A)5) were deleted from an N-terminus side to a C-terminus side, and one [(A)n motif-REP] was inserted in front of the C-terminus sequence, and all GGX in REP were substituted with GQX. The amino acid sequence shown in SEQ ID NO: 10 was obtained by adding the amino acid sequence (tag sequence and hinge sequence) shown in SEQ ID NO: 11 to the N-terminus of the amino acid sequence shown in SEQ ID NO: 9.

A nucleic acid encoding protein having the amino acid sequence shown in SEQ ID NO: 10 obtained by adding the His tag sequence and the hinge sequence (SEQ ID NO: 11) to the N-terminus of the amino acid sequence shown in SEQ ID NO: 9 was synthesized. In the nucleic acid, an NdeI site was added at a 5' end and an EcoRI site was added downstream of a stop codon. The nucleic acid was cloned into a cloning vector (pUC118). Thereafter, the same nucleic acid was cut out by restriction enzyme treatment with NdeI and EcoRI, and then recombined into a protein expression vector pET-22b (+) to obtain an expression vector.

### (2) Protein Expression

E. coli BLR (DE3) was transformed with the pET 22b (+) expression vector including the nucleic acid encoding protein having the amino acid sequence shown in SEQ ID NO: 10. The transformed E. coli was cultured in 2 mL of LB medium containing ampicillin for 15 hours. The same culture solution was added to 100 mL of seed culture medium (Table 1) containing ampicillin such that an OD₆₀₀ became 0.005, to be inoculated with the transformed E. coli. A temperature of the culture solution was kept at 30°C, and flask culture was performed until the OD₆₀₀ became 5 (approximately 15 hours) to obtain a seed culture solution.

### [Table 1]

**Table 1. Seed culture medium (at the time of starting culturing, per 1 L)**

| | |
|---|---|
| Glucose | 5 g |
| KH₂PO₄ | 4 g |
| K₂HPO₄ | 10 g |
| Yeast Extract | 6 g |

| | |
|---|---|
| Ampicillin was added, such that a final concentration became 100 mg/mL, to obtain a seed culture medium. | |

The seed culture solution was added to 500 ml of production medium (Table 2) such that the OD₆₀₀ became 0.05, to be inoculated with the transformed E. coli. The temperature of the culture solution was kept at 37°C, and culturing was performed by controlling a pH to be constant at 6.9. In addition, a dissolved oxygen concentration in the culture solution was maintained at 20% of a dissolved oxygen saturation concentration.

### [Table 2]

**Table 2. Production medium (at the time of starting culturing, per 1 L)**

| | |
|---|---|
| Glucose | 12 g |
| KH₂PO₄ | 9 g |
| MgSO₄·7H₂O | 2.4 g |
| Yeast Extract | 15 g |
| FeSO₄·7H₂O | 40 mg |
| MnSO₄·5H₂O | 40 mg |
| CeCl₂·2H₂O | 40 mg |
| GD-113 (Antifoaming agent) | 0.1 mL |

Immediately after glucose in the production medium was completely consumed, a feed solution (glucose 455 g/1 L, Yeast Extract 120 g/1 L) was added at a rate of 1 ml/min. The temperature of the culture solution was kept at 37°C, and culturing was performed by controlling a pH to be constant at 6.9. In addition, the dissolved oxygen concentration in the culture solution was maintained at 20% of the dissolved oxygen saturation concentration, and culturing was performed for 20 hours. Thereafter, 1 M isopropyl-β-thiogalactopyranoside (IPTG) was added to the culture solution such that a final concentration became 1 mM, to induce expression of the target protein. At the time when 20 hours elapsed after the IPTG addition, the culture solution was centrifuged to recover fungus bodies. The SDS-PAGE was performed using the fungus bodies prepared from the culture solution before the IPTG addition and after the IPTG addition, and expression of target protein was confirmed by appearance of a band of a target protein size depending on the IPTG addition.

### (3) Purification of Protein

Fungus bodies recovered after 2 hours from the IPTG addition were washed with 20 mM Tris-HCl buffer (pH 7.4). The fungus bodies after washed were suspended in 20 mM Tris-HCl buffer solution (pH 7.4) containing approximately 1 mM PMSF, and the cells were disrupted with a high-pressure homogenizer (GEANiro Soavi). The disrupted cells were centrifuged to obtain a precipitate. The resulting precipitate was washed with 20 mM Tris-HCl buffer solution (pH 7.4) to reach high purity. The precipitate after washed was suspended in 8 M guanidine buffer solution (8 M guanidine hydrochloride, 10 mM sodium dihydrogen phosphate, 20 mM NaCl, 1 mM Tris-HCl, and pH 7.0) such that a concentration became 100 mg/mL, and was stirred at 60°C for 30 minutes with a stirrer to be dissolved. After dissolution, dialysis was performed with water by using a dialysis tube (cellulose tube 36/32 manufactured by Sanko Junyaku Co., Ltd.). White aggregated protein obtained after the dialysis was recovered by centrifugation, water was removed by a lyophilizer, and the lyophilized powder was recovered.

A degree of purification of the target protein in the obtained lyophilized powder was confirmed by image analysis of a result of polyacrylamide gel electrophoresis of the powder by using Total lab (nonlinear dynamics ltd.). As a result, the degree of purification of the protein was approximately 85%.

### [Recovery of Spider Silk Protein]

### (Example 1)

A knit obtained by inter-knitting (mixing and knitting) the spider silk protein obtained above (artificial spider silk fiber, hereinafter also referred to as "SSP") and a wool fiber which is a material different from the spider silk protein (hereinafter, also referred to as "other material") was cut into approximately 2 cm square and approximately 1.79 g of knit was prepared. The respective weights of the spider silk protein and the wool fiber were 0.41 g and 1.38 g, by calculation. The knit and 6.78 g (water 4.75 g, CLYNSOLVE P-7 2.03 g) of mixed solvent of water as a polar solvent and CLYNSOLVE P-7 (a mixed solvent containing 85.5±1.0% of ethanol, less than 5.0% of isopropanol, 9.6±0.5% of normal propanol, and 0.2% or less of water, "CLYNSOLVE" is a registered trademark of Japan Alcohol Trading CO., LTD) (Water:CLYNSOLVE P-7=7:3) were input to a container with a stirrer and the container was sealed.

The container was set to a hot stirrer (manufactured by TOKYO RIKAKIKAI CO., LTD., RCH-20L), a heater temperature was set to 90°C, and stirring was performed at 300 rpm. The contents reached approximately 90°C in first 10 minutes, and at the same time, internal pressure reached 0.08 MPa, by calculation, due to the vapor pressure of the solvent. From this state, stirring was further continued for 20 minutes. Thereafter, the container was removed from the hot stirrer and allowed to stand at a room temperature until the temperature fell to 70°C, which is equal to or lower than a boiling point of the solvent.

A lid of the container was detached, the stirrer was removed. Then, contents of the container were separated into a solution and insoluble matter by using a filter. Further, the insoluble matter was washed with water in the container, separated into a solution and insoluble matter by using a similar filter. This operation was repeated three times.

All filtered solutions were transferred to the same container and separated from the insoluble matter. The resulting solution and the insoluble matter were dried at 80°C for 12 hours using a thermostat (PVH-212M manufactured by ESPEC Corp.), respectively.

After drying, respective weights of the solid matters obtained from the solution and the insoluble matter was measured. Results of respective weights of the spider silk protein and the wool fiber in the inter-knitted knit (before the treatment), respective weights thereof after the treatment, and yields thereof are shown in Table 3.

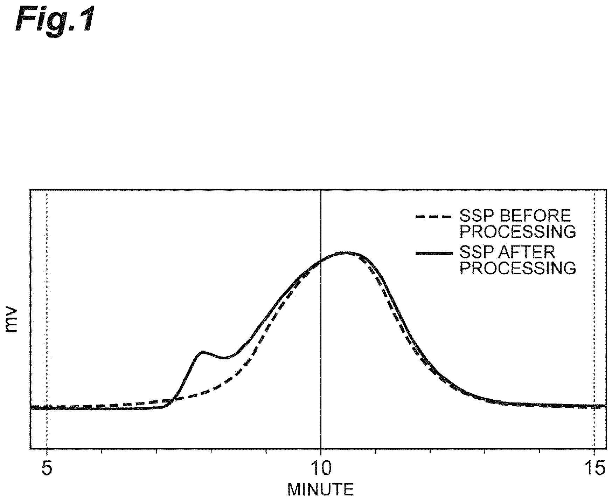

In addition, FIG. 1 shows results of GPC measurement of the spider silk protein before and after the treatment. As a result, since the results of the GPC measurement of the spider silk protein before and after the treatment were almost the same as each other, it was determined that the spider silk protein could be recovered almost as it was. In a graph after the treatment, presence of a substance is observed on a high molecular weight side (left side of the graph in FIG. 1), but it is considered that because the spider silk protein is aggregated by ethanol.

For the GPC measurement, GPC (manufactured by Shimadzu Corporation, trade name: 2C-20AD), column LF-404 (manufactured by Showa Denko), Shodex detector (manufactured by Showa Denko, trade name: RI-504) were used.

### (Example 2)

The wool fiber in Example 1 was changed to a cotton fiber, and the same experiment as in Example 1 was performed. Results thereof are shown in Table 3.

### [Table 3]

**Table 3. Polar solvent: Mixed solvent of water and alcohol**

| | Another material | Used weight/g | Used weight/g (Calculated value) | | Weight after treatment/g | Yield/% |
|---|---|---|---|---|---|---|
| Example 1 | Wool | 1.79 | SSP | 0.41 | 0.15 | 37.9% |
| | | | Wool | 1.38 | 1.58 | 114.5% |
| Example 2 | Cotton | 1.79 | SSP | 0.43 | 0.22 | 51.9% |
| | | | Cotton | 1.35 | 1.48 | 109.4% |

As described in Table 3, it was shown that, in a case where the mixed solvent of water and alcohol was used as the polar solvent, the SSP was recovered by applying pressure while heating the polar solvent containing the mixture of the SSP and another material. In addition, although the weight of the other material after the treatment is larger than that before processing, it is considered that a cause is that a part of the spider silk protein remains as attached.

### (Example 3)

The polar solvent in Example 1 was changed to water and the set temperature of the hot stirrer was changed to 110°C, and the same experiment as in Example 1 was performed. The contents reached approximately 110°C in approximately 10 minutes from the start of the stirring, and at the same time, the internal pressure reached 0.15 MPa, by calculation, due to the vapor pressure of the solvent. In addition, the container was removed from the hot stirrer after the stirring, and thereafter, it was allowed to stand at a room temperature until the temperature of the container fell to 90°C, which is equal to or lower than a boiling point of the solvent. Results of respective weights of the spider silk protein and the cotton fiber in the inter-knitted knit (before the treatment), respective weights thereof after the treatment, and yields thereof are shown in Table 4.

### (Example 4)

The wool in Example 3 was changed to cotton, and the same experiment as in Example 3 was performed. Results thereof are shown in Table 4.

### [Table 4]

**Table 4. Polar solvent: Water**

| | Another material | Used weight/g | Used weight/g (Calculated value) | | Weight after treatment/g | Yield/% |
|---|---|---|---|---|---|---|
| Example 3 | Wool | 1.79 | SSP | 0.41 | 0.09 | 21.2% |
| | | | Wool | 1.38 | 1.67 | 120.8% |
| Example 4 | Cotton | 1.80 | SSP | 0.43 | 0.27 | 63.0% |
| | | | Cotton | 1.36 | 1.46 | 107.5% |

As described in Table 4, even in a case where water was used as the polar solvent, it was shown that the SSP was recovered from the mixture including the SSP and another material (wool or cotton).

Hereinafter, a similar experiment using a spider silk protein powder and a woven fabric of another fiber will be briefly described, instead of the knit obtained by inter-knitting the spider silk protein fiber and another fiber.

### (Example 5)

A plain-woven non-colored nylon fabric was cut into approximately 2 cm square and approximately 0.5 g of nylon fabric was prepared. This nylon fabric, 0.5 g of spider silk protein powder, and 4.5 g (water 3.15 g, CLYNSOLVE P-7 1.35 g) of a mixed solvent of water as a polar solvent and CLYNSOLVE (registered trademark) P-7 (Water:CLYNSOLVE P-7=7:3) were input to a container with a stirrer and the container was sealed.

The container was set to a hot stirrer (manufactured by TOKYO RIKAKIKAI CO., LTD., RCH-20L), a heater temperature was set to 90°C, and stirring was performed at 300 rpm. The contents reached approximately 90°C in first 10 minutes, and at the same time, internal pressure reached 0.08 MPa, by calculation, due to the vapor pressure of the solvent. From this state, stirring was further continued for 20 minutes. Thereafter, the container was removed from the hot stirrer and allowed to stand at a room temperature until the temperature fell to 70°C, which is equal to or lower than a boiling point of the solvent.

A lid of the container was detached, the stirrer was removed. Then, contents of the container were separated into a solution and insoluble matter by using a filter. Further, the insoluble matter was washed with water in the container, separated into a solution and insoluble matter by using a similar filter. This operation was repeated three times.

All filtered solutions were transferred to the same container and separated from the insoluble matter. The resulting solution and the insoluble matter were dried at 80°C for 12 hours using a thermostat (PVH-212M manufactured by ESPEC Corp.), respectively.

After drying, respective weights of the solid matters obtained from the solution and the insoluble matter was measured. Results of respective weights of the spider silk protein and the mixture (before the treatment) of the nylon fabric, respective weights thereof after the treatment, and yields thereof are shown in Table 5.

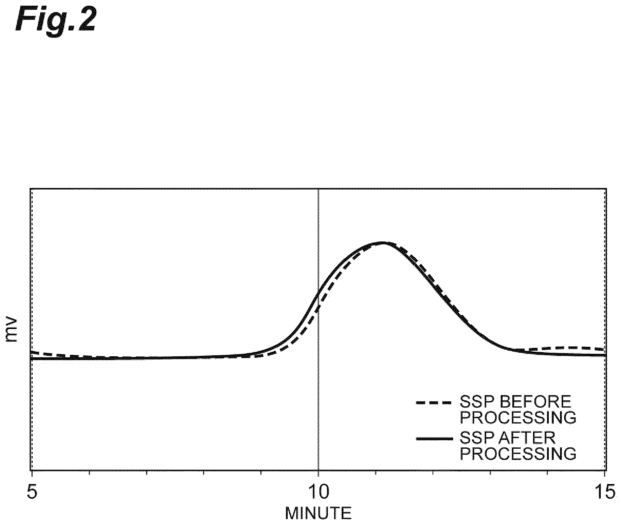

In addition, FIG. 2 shows results of GPC measurement of the spider silk protein before and after the treatment. As a result, since the results of the GPC measurement of the spider silk protein before and after the treatment were almost the same as each other, it was determined that the spider silk protein could be recovered almost as it was. The GPC measurement was carried out under the same conditions as in Example 1.

### (Example 6)

The non-colored nylon fabric in Example 5 was changed to a nylon fabric dyed in red, and the same experiment as in Example 5 was performed. Results thereof are shown in Table 5.

### (Example 7)

The non-colored nylon fabric in Example 5 was changed to a PET fabric dyed in blue, and the same experiment as in Example 5 was performed. Results thereof are shown in Table 5.

### (Example 8)

The non-colored nylon fabric in Example 5 was changed to a non-colored PET fabric, and the same experiment as in Example 5 was performed. Results thereof are shown in Table 5.

### [Table 5]

**Table 5. Polar solvent: Mixed solvent of water and alcohol**

| | Another material | | Used weight/g (Calculated value) | | Weight after treatment/g | Yield/% |
|---|---|---|---|---|---|---|
| Example 5 | Nylon | Non-colored | SSP | 0.50 | 0.49 | 96.9% |
| | | | Nylon | 0.51 | 0.50 | 99.3% |
| Example 6 | Nylon | Red | SSP | 0.50 | 0.40 | 79.9% |
| | | | Nylon | 0.51 | 0.54 | 105.9% |
| Example 7 | PET | Blue | SSP | 0.51 | 0.47 | 93.7% |
| | | | PET | 0.51 | 0.55 | 107.8% |
| Example 8 | PET | Non-colored | SSP | 0.51 | 0.49 | 95.9% |
| | | | PET | 0.50 | 0.52 | 103.3% |

As described in Table 5, it was shown that, in a case where the mixed solvent of water and alcohol was used as the polar solvent, even when the other material was any of the nylon and the PET, the spider silk protein can be recovered by applying pressure while heating the polar solvent containing the mixture of the SSP and another material. In addition, it was also shown that, even in a case of using another material dyed in red or blue, the spider silk protein was recovered.

### (Example 9)

The solvent in Example 5 was changed to water and the set temperature of the hot stirrer was changed to 110°C, and the same experiment as in Example 5 was performed. The contents reached approximately 110°C in approximately 10 minutes from the start of the stirring, and at the same time, the internal pressure reached 0.15 MPa, by calculation, due to the vapor pressure of the solvent. In addition, the container was removed from the hot stirrer after the stirring, and thereafter, it was allowed to stand at a room temperature until the temperature of the container fell to 90°C, which is equal to or lower than a boiling point of the solvent. Results of respective weights of the spider silk protein and the mixture of the nylon fabric, respective weights thereof after the treatment, and yields thereof are shown in Table 6.

### (Example 10)

The non-colored nylon fabric in Example 9 was changed to a nylon fabric dyed in red, and the same experiment as in Example 9 was performed. Results thereof are shown in Table 6.

### (Example 11)

The non-colored nylon fabric in Example 9 was changed to a PET fabric dyed in blue, and the same experiment as in Example 9 was performed. Results are shown in Table 6.

### (Example 12)

The non-colored nylon fabric in Example 9 was changed to a non-colored PET fabric, and the same experiment as in Example 9 was performed. Results thereof are shown in Table 6.

### [Table 6]

**Table 4. Polar solvent: Water**

| | Another material | | Used weight/g (Calculated value) | | Weight after treatment/g | Yield/% |
|---|---|---|---|---|---|---|
| Example 9 | Nylon | Non-colored | SSP | 0.50 | 0.47 | 94.7% |
| | | | Nylon | 0.51 | 0.52 | 101.0% |
| Example 10 | Nylon | Red | SSP | 0.50 | 0.41 | 81.2% |
| | | | Nylon | 0.50 | 0.59 | 116.9% |
| Example 11 | PET | Blue | SSP | 0.51 | 0.27 | 52.7% |
| | | | PET | 0.51 | 0.60 | 118.0% |
| Example 12 | PET | Non-colored | SSP | 0.51 | 0.29 | 57.5% |
| | | | PET | 0.51 | 0.62 | 121.7% |

As described in Table 6, it was shown that, even in a case of using water as the polar solvent and nylon or PET as another material, the spider silk protein can be recovered. In addition, it was also shown that, even in a case of using another material dyed in red or blue, the spider silk protein was recovered.

## Claims

1. A method for recovering protein, which recovers a target prote in from a mixture containing the target protein and a material diff erent from the target protein, the method comprising:
a dissolution step of dissolving either the target protein or the material by applying pressure while heating to a solution for dissolution containing the mixture and a polar solvent; and
a separation step of separating an obtained solution.

2. The method for recovering protein according to claim 1,
wherein the dissolution step is a step of dissolving the tar get protein.

3. The method for recovering protein according to claim 1 or 2,
wherein the target protein is one or more proteins selected from the group consisting of silk fibroin, spider silk fibroin, and hornet silk fibroin.

4. The method for recovering protein according to any one of cl aims 1 to 3,
wherein the polar solvent includes one or more solvents s elected from the group consisting of water, alcohol, dimethyl sulfo xide, dimethylformamide, and hexafluoroacetone.

5. The method for recovering protein according to any one of claims 1 to 4,
wherein the material includes one or more materials selected from the group consisting of polyester, nylon, cotton, and wool.
